# EUROPÄISCHE PATENTSCHRIFT

(11) **EP 0 950 653 B1**
(45) Veröffentlichungstag und Bekanntmachung des Hinweises auf die Patenterteilung: **21.11.2001**
(21) Anmeldenummer: 99104526.1
(22) Anmeldetag: 06.03.1999
(51) Int. Cl.: C07C 69/38, C07C 67/30

(54) **Verfahren zur Herstellung von Kaliummonoethylmalonat**
Process for the preparation of potassium monoethylmalonate
Procédé de préparation du malonate de monoéthyle et de potassium

(30) Priorität: 17.04.1998 DE 19817101
(43) Veröffentlichungstag der Anmeldung: 20.10.1999
(73) Patentinhaber: Degussa AG, 40474 Düsseldorf (DE)
(72) Erfinder: Muhr, Jürgen Dr., 53347 Alfter (DE); Feld, Marcel Dr., 51147 Köln (DE)

(56) Entgegenhaltungen:
- WO-A-97/10243
- US-A- 3 855 229

## Beschreibung

Die Erfindung betrifft ein Verfahren zur Herstellung von Kaliunmonoethylmalonat (oder des Kaliumsalzes des Malonsäuremonoethylesters: nachfolgend als KEM bezeichnet) durch selektive Verseifung von Diethylmalonat (oder des Malonsäurediethylesters; nachfolgend als DEM bezeichnet) mit Kaliumhydroxid, wobei das Zielprodukt in hoher Reinheit gewonnen wird.

KEM wird als Vorprodukt für die Synthese von Pharmawirkstoffen mit Chinolonstruktur verwendet. Der Einsatz für Synthesen im Pharmabereich stellt allerdings hohe Ansprüche an die Reinheit des Produktes. Insbesondere ist ein erheblicher Gehalt an Dikaliummalonat (nachfolgend als DKM bezeichnet) unerwünscht.

Eine schon im vergangenen Jahrhundert von van't Hoff (Ber.Dtsch.Chem. Ges., 7. 1572) beschriebene und auch heute noch praktizierte Synthese für Kaliummonoethylmalonat geht von DEM aus, das mit Kaliumhydroxid selektiv verseift wird. Gemäß EP 0 720 981 A1 erfolgt dies unter Einsatz äquimolarer Eduktmengen in alkoholischem Medium. Die angestrebte selektive Verseifung wird allerdings nur in unzureichendem Maße erreicht, so daß man lediglich ein mit DKM erheblich verunreinigtes Zielprodukt erhält. Der Gehalt an DKM liegt üblicherweise in der Größenordnung von mehreren Gew.-%. Die Abtrennung des Dikaliummalonats aus dem Kaliummonoethylmalonat. die für die Verwendung des letzteren als Pharmavorprodukt erforderlich ist, ist schwierig und schließt umfangreiche Reinigungsoperationen ein. Ein weiterer Nachteil des bekannten Verfahrens besteht in der relativ starken Verdünnung, in der das Verfahren durchgeführt werden muß. Der Alkohol wird in der 19-bis 28-fachen Gewichtsmenge, bezogen auf Kaliumhydroxid, eingesetzt. Diese große Alkoholmenge wirkt sich nachteilig auf die Raum-Zeit-Ausbeute aus und erhöht den Aufwand für die Rückgewinnung des Lösemittels. Bei den erwähnten Verfahren wird die Alkoholmenge weiterhin dadurch erhöht. daß auch das DEM in alkoholischer Lösung eingesetzt wird.

Box et al., Heterocycles, Vol. 32, No. 2, 1991, 245 ff., erwähnen eine Synthese von β-Lactonen und β-Lactamen, bei der KEM als Zwischenprodukt dient. Im experimentellen Teil wird auf Seite 247 die Herstellung von KEM aus DEM und KOH beschrieben. Die beiden Edukte werden, wie bei dem Verfahren der erwähnten EP 0 720 981 A1, in äquimolaren Mengen eingesetzt, nämlich jeweils 100 mmol. Die molaren Verhältnisse von DEM zu KOH wurden jedoch falsch berechnet, denn 20.225 g DEM sind 126 mmol und entsprechen der 1,26-fachen molaren Menge. bezogen auf KOH. Wenn man weiterhin annimmt, daß das eingesetzte Kaliumhydroxid. wie handelsüblich, einen KOH-Gehalt von ca. 90 Gew.-% (Rest Wasser) hatte, entsprechen 20,225 g DEM sogar der 1,38-fachen molaren Menge, bezogen auf KOH (gerechnet 100%). Eine Nacharbeitung mit molaren Verhältnissen von 1,26:1 und von 1,38:1 ergab, daß nach diesem Verfahren zwar ein reines KEM mit weniger als 0,5 Gew.-% DKM erhältlich ist, das ausgefallene KEM jedoch sehr schlecht filtrierbar war. Bei Laboransätzen betrug die Filtrierzeit mehr als zwei Stunden. Solche Zeiten sind für eine Produktion im technischen Maßstab prohibitiv. Zudem werden nach Box et al., ähnlich wie bei dem Verfahren der erwähnten EP 0720 981 A1, erhebliche Mengen an Alkohol benötigt. Schließlich sind auch die Ausbeuten an KEM nicht befriedigend.

Eine der Aufgaben der vorliegenden Erfindung ist die Bereitstellung eines Verfahrens zur Herstellung von Kaliummonoethylmalonat, das die genannten Nachteile vermeidet, d.h. ohne Mitverwendung von großen Mengen an Alkohol hohe Ausbeuten an einem reinen, DKM-armen und leicht filtrierbaren KEM ergibt.

Diese Aufgabe wurde erfindungsgemäß gelöst durch ein Verfahren zur Herstellung von Kaliummonoethylmalonat durch selektive Verseifung von Malonsäurediethylester mit Kaliumhydroxid, bei dem man das Kaliumhydroxid dem Malonsäurediethylester zufügt. Malonsäurediethylester und Kaliumhydroxid in einem molaren Verhältnis von mindestens 1.5 anwendet und das Kaliumhydroxid in dem Malonsäurediethylester wirkungsvoll verteilt.

Das erfindungsgemäße Verfahren ergibt Kaliummonoethylmalonat mit einem Gehalt an DKM von <1 Gew.-%. wie es für Pharmasynthesen benötigt wird, zeigt akzeptable Filtrierzeiten und erfordert einen vergleichsweise geringen Energieaufwand zur Rückgewinnung des Alkohols und des überschüssigen Malonsäurediethylesters. Die hohe Reinheit des KEM war überraschend, denn die erfindungsgemäßen Maßnahmen zeigen im Falle des homologen Malonsäuredimethylesters (oder Dimethylmalonats: DMM) keine vergleichbare Wirkung. Aus den Vergleichsbeispielen 2 und 3 geht hervor, daß DMM auch ohne das erfindungsgemäße Molverhältnis von KOH zu DEM und auch ohne wirkungsvolle Verteilung des Kaliumhydroxids ein Kaliummonomethylmalonat (KMM) mit niedrigen Gehalten an DKM ergibt. Weiterhin war überraschend und ist für die Produktion im technischen Maßstab entscheidend, daß das KEM erfindungsgemäß sehr gut filtrierbar ist, wie aus dem Vergleichsbeispiel 6 ersichtlich ist. Dieser Effekt ist auch im Nachhinein nicht erklärbar. Schließlich ist überraschend, daß das dem KEM analoge Natriummonoethylmalonat (NaEM) unter den erfindungsgemäßen Bedingungen ein NaEM mit hohem Gehalt an Dinatriummalonat (DNaM) ergibt, noch dazu in schlechter Ausbeute, wie das Vergleichsbeispiel 4 zeigt. Hinzu kommt, daß das NaEM-Reaktionsgemisch erheblich schlechter filtrierbar ist als das entsprechende KEM-Gemisch nach dem erfindungsgemäßen Verfahren. Die Filtration des ersteren dauert ca. zehnmal länger, mit entsprechend schlechter Raum-Zeit-Ausbeute.

Ein wesentliches Merkmal des erfindungsgemäßen Verfahrens besteht darin, daß das Kaliumhydroxid dem Malonsäurediethylester zugefügt wird. der also im gesamten Reaktionsverlauf im Überschuß vorliegt. 0as Kaliumhydroxid wird vorteilhaft in Form einer Lösung, insbesondere einer alkoholischen Lösung angewandt. Um Umesterungen zu vermeiden. verwendet man zweckmäßig Ethanol als alkoholisches Lösemittel. Man kann dazu das ca. 96-gewichtsprozentige Ethanol einsetzen, wie es als Azeotrop bei der Destillation von wäßrig-alkoholischen Gemischen anfällt. Alternativ kann man Ethanol mit anderen, ähnlich geringen Wassergehalten oder vorzugsweise den sog. absoluten, wasserfreien Alkohol verwenden. Die Lösung kann weitere. inerte niedrigsiedende Lösemittel enthalten, wie aliphatische, cycloaliphatische oder aromatische Kohlenwasserstoffe, ohne daß damit ein besonderer Vorteil verbunden wäre. In der Lösung liegen der Alkohol und gegebenenfalls weitere Lösemittel in der Regel in der 3- bis 10-fachen, vorteilhaft in der 4 bis 8-fachen Gewichtsmenge, bezogen auf Kaliumhydroxid vor. Das Verfahren nach der Erfindung kommt also mit weniger Alkohol bzw. Lösemittel aus als die zuvor erwähnten Verfahren nach dem Stand der Technik.

Ein weiteres wesentliches Merkmal des erfindungsgemäßen Verfahrens ist der molare Überschuß, in dem der Malonsäurediethylester eingesetzt wird, und zwar vorteilhaft in Substanz, also ohne Löse- oder Verdünnungsmittel. Mit einem Molverhältnis von 1,5 bis 12 Mol, vorzugsweise von 2 bis 8 Mol und insbesondere von 3 bis 5 Mol Malonsäurediethylester je Mol Kaliumhydroxid erreicht man einerseits die hohe Produktreinheit, die für das erfindungsgemäße Verfahren kennzeichnend ist, und stellt andererseits sicher, daß das Reaktionsgemisch trotz des kristallin ausfallenden Zielproduktes als Suspension handhabban bleibt. Der Überschuß Kann innerhalb der angegebenen Grenzen geringer sein, wenn die Menge des für die Kaliumhydroxidlösung eingesetzten Alkohols bzw. weiteren Lösemittels innerhalb der hierfür angegebenen Grenzen im oberen Bereich liegt. Der überschüssige Malonsäurediethylester kann durch Destillation praktisch vollständig zurückgewonnen werden.

Ein drittes wesentliches Merkmal der Erfindung ist die wirkungsvolle Verteilung (oder gute Durchmischung) des zugefügten Kaliumhydroxids in dem überschüssigen Malonsäurediethylester durch Einwirkung von Scherkräften auf das Gemisch. Dies kann z.B. durch besonders effektive Rührung und/oder durch Umpumpen des Reaktionsgemisches, gegebenenfalls durch Rohre mit die Mischung fördernden Einbauten und/oder durch Mischdüsen, erreicht werden. Das Merkmal "wirkungsvolle Verteilung" eignet sich nicht zu einer quantitativen Beschreibung. 0urch orientierende Versuche läßt sich die optimale Verteilung (oder wirkungsvolle Durchmischung) unschwer ermitteln. Sie ist dann gegeben. wenn eine Erhöhung des Energieeintrags unter sonst gleichen Bedingungen keine Verbesserung der Ausbeute und/oder der Produktqualität ergibt.

Das erfindungsgemäße Verfahren wird vorteilhaft bei Temperaturen von <80°C, vorzugsweise von <60°C und insbesondere von 0°C bis etwa 30°C durchgeführt. Unter diesen Bedingungen läuft die partielle Verseifung des Malonsäurediethylesters rasch und selektiv ab. Die Reaktion ist praktisch beendet, wenn das gesamte Kaliumhydroxid zugeführt worden ist, eine längere Nachreaktionszeit ist nicht erforderlich. Nach Beendigung der Reaktion kann die Temperatur gesteigert werden, gegebenenfalls unter Abdestillieren niedrigsiedender Anteile, wie Ethanol oder weiterer niedrigsiedender Lösemittel.

Das Verfahren nach der Erfindung kann beispielsweise diskontinuierlich durchgeführt werden, indem man den Malonsäurediethylester bei Raumtemperatur in einem Rührreaktor vorlegt und eine möglichst wasserarme oder wasserfreie Lösung von Kaliumhydroxid in Ethanol unter intensiver Durchmischung innerhalb von 30 Minuten bis 4 Stunden nach und nach zufließen läßt. Dabei bestimmt die Intensität der Durchmischung das Tempo, in dem die Kaliumhydroxidlösung zugeführt wird. Der Malonsäurediethylester kann unverdünnt eingesetzt werden oder Lösemittelanteile enthalten, beispielsweise wenn rückgewonnener überschüssiger Malonsäurediethylester verwendet oder mitverwendet wird. Je nach der Menge der eingesetzten Edukte bleibt die Temperatur ohne weitere Maßnahmen in dem angegebenen Bereich oder sorgt man durch indirekte Kühlung dafür, daß die Temperatur in diesem Bereich bleibt.

Bei einer kontinuierlichen Ausführungsform des Verfahrens dosiert man die Kaliumhydroxidlösung in den überschüssigen Malonsäurediethylester und führt das Gemisch über eine Verteilerdüse in eine Mischstrecke. in der die partielle Verseifung innerhalb von Sekunden bis zu wenigen Minuten abläuft.

Das Zielprodukt Kaliummonoethylmalonat fällt in fester Form an und kann nach beendeter Reaktion, gegebenenfalls auch kontinuierlich. durch eine übliche Fest/Flüssig-Trennung abgetrennt werden. beispielsweise mittels einer Nutsche, Filterpresse oder Zentrifuge, Durch Auswaschen mit einem niedrigsiedenden Lösemittel, zweckmäßig einem Alkohol, entfernt man anhaftenden überschüssigen Malonsäurediethylester. Es ist ein weiterer Vorteil des Verfahrens nach der Erfindung, daß das Kaliummonoethylmalonat in einer leicht filtrierbaren und gut auswaschbaren Form anfällt, was ebenfalls zu der hohen Produktreinheit und dem vergleichsweise niedrigen Lösemittelbedarf beiträgt. Die als Filtrat erhaltene Mutterlauge sowie die Maschflüssigkeit können nach Abtrennung des bei der partiellen Verseifung gebildeten bzw. mit dem Kaliumhydroxid eingebrachten Alkohols sowie gegebenenfalls zusätzlich in die Reaktion eingeführter Lösemittel und nach Ergänzung des verbrauchten Malonsäurediethylesters als Edukt und Reaktionsmedium in das Verfahren zurückgeführt, d.h. für einen neuen diskontinuierlichen Ansatz verwendet oder in das kontinuierliche Verfahren zurückgeführt werden. Der Alkohol und die Lösemittel können unter schonenden Bedingungen, d.h. unter vermindertem Druck und/oder unter Mitverwendung eines Azeotrop-bildenden inerten Schleppmittels. abgetrennt werden. Dadurch wirkt man der Entstehung unerwünschter Nebenprodukte entgegen. Als Beispiele für inerte Schleppmittel, die das Abdestillieren des Alkohols erleichtern, seien insbesondere aliphatische und/oder aromatische Kohlenwasserstoffe genannt, die nach beendeter Reaktion oder schon während oder vor der Reaktion zugesetzt werden können.

Die folgenden Beispiele sollen die Erfindung weiter erläutern, nicht aber ihren Anwendungsbereich begrenzen.

### Beispiel 1 - Herstellung von KEM

Eine Lösung von 111,1g KOH (91-gew.-%ig; 1.8 mol) in 500g 98%-igem Ethanol wird bei 15-20°C innerhalb von etwa 2h unter intensiver Durchmischung zu 900g DEM (5.6 mol) dosiert. Die Durchmischung erfolgt mittels eines KPG-Rührers, der mit 300 U/min betrieben wird. Die entstandene Suspension wird abfiltriert. Die Filtration mittels Nutsche und Wasserstrahlvakuum nimmt ca. 3 min in Anspruch. Der Filterrückstand wird mit Ethanol ausgewaschen und im Vakuum getrocknet. Man erhält 261,9g KEM, entsprechend 85,5 % d.Th.. bezogen auf KOH. Der Gehalt an DKM beträgt 0,3 Gew.-%. Er wurde hier und in den folgenden Beispielen durch Ionenchromatographie bestimmt.

Aus dem Filtrat und der Waschflüssigkeit werden durch Destillation die Leichtsieder zurückgewonnen. Der Sumpfrückstand enthält das überschüssige DEM sowie gelöst gebliebenes bzw. beim Auswaschen wieder gelöstes KEM. Man ersetzt durch Umsetzung verbrauchtes DEM und dosiert in einem neuen Ansatz KOH-Lösung zu, die aus den rückgewonnenen Leichtsiedern bereitet wird. Auf diese Weise erzielt man nach mehreren Recyclisierungen KEM mit einer mittleren Ausbeute von etwa 95% d.Th. und einem mittleren DKM-Gehalt von <0.6 Gew.-%.

### Vergleichsbeispiel 1 - Herstellung von Kaliummonoethylmalonat

Man arbeitet wie im Beispiel 1, legt aber nur 320,4 g DEM (2.1 mol) vor und betreibt den KPG-Rührer mit 50 U/min. Man erhält 252.5 g KEM. entsprechend 82,6 % d.Th., mit einem Gehalt an DKM von >5 Gew.-%.

Aus dem Beispiel 1 und dem Vergleichsbeispiel 1 geht hervor, daß die Kombination von überschüssigem DEM mit guter Durchmischung die Ausbeute und die Produktqualität verbessert.

### Vergleichsbeispiel 2 - Herstellung von Kaliummonomethylmalonat (KMM)

111.6g KOH (1.8 mol) werden in 500 g Methanol gelöst, und die Lösung wird innerhalb von 2h bei Raumtemperatur zu 264,2g Dimethylmalonat (2 mol) dosiert. Dabei wird der KPG-Rührer mit 50 U/min betrieben. Dadurch wird die Reaktionssuspension unzulänglich durchmischt, breite Randzonen bleiben nahezu unvermischt. Anschließend wird die Reaktionssuspension filtriert und der Filterrückstand mit Methanol gewaschen und im Vakuum getrocknet. Man erhält 214.2 g KMM. entsprechend einer Ausbeute von 76 % d.Th. mit einem Gehalt an DKM von <0.2 Gew.-%.

### Vergleichsbeispiel 3 - Herstellung von Kaliummonomethylmalonat

Man arbeitet wie im Vergleichsbeispiel 1, betreibt aber den KPG-Rührer mit 200 U/min und erhält 217,8g KMM, entsprechend 77,5 % d.Th.. mit einem DKM-Gehalt von <0,2 Gew.-%.

Aus den Vergleichsbeispielen 2 und 3 geht hervor, daß bei der Herstellung von KMM die Art der Durchmischung ohne Einfluß auf die Ausbeute und die Produktqualität ist.

### Vergleichsbeispiel 4 - Herstellung von Natriummonoethylmalonat (NaME)

Man arbeitet wie im Beispiel 1, setzt jedoch an Stelle der Kalilauge 429g 12.6-gewichtsprozentige Natronlauge (1,4 mol NaOH) ein. Man erhält 39,0g NaME, entsprechend 15.2% d.Th., mit einem Gehalt an Dinatriummalonat (DNaM) von 18.6%.

Das Vergleichsbeispiel zeigt, daß ein Überschuß an DEM sowie gute Durchmischung beim NaME nicht zu den Ausbeuten und der Produktqualität führen, die bei KEM erzielt werden.

### Vergleichsbeispiel 5 - Herstellung von Kaliummonoethylmalonat (KMM)

28.2g KOH (91 gew.-%-ig: 0,46 mol) werden in 500ml 98%igem Ethanol gelöst. Die Lösung wird tropfenweise innerhalb 1h einer mittels KPG-Rührer (250 U/min) gerührten Lösung von 100,9g DEM (0,63mol) in 500ml 98%-igem Ethanol zugesetzt, wobei die Temperatur auf 0°C gehalten wurde. Man ließ das Reaktionsgemisch sich auf Raumtemperatur erwärmen und rührte es eine weitere Stunde lang. die ausgefallenen Kristalle wurden durch Vakuumfiltration (Wasserstrahlpumpenvakuum) abgetrennt und mit 98%-igem Alkohol gewaschen. Die Filtration nahm 2,3h in Anspruch. Man erhielt 53,3 g KEM, entsprechend 68,8% d.Th., mit einem DKM-Gehalt von 0,1 Gew.-%

Das Vergleichsbeispiel zeigt, daß die Arbeitsweise nach Box et al. (a.a.O.) zwar ein sehr reines KEM ergibt, aber in unbefriedigenden Ausbeuten, noch dazu unter Einsatz großer Mengen an Ethanol und mit langen Filtrierzeiten.

## Patentansprüche

1. Verfahren zur Herstellung von Kaliummonoethylmalonat durch selektive Verseifung von Malonsäurediethylester mit Kaliumhydroxid, **dadurch gekennzeichnet, daß** man das Kaliumhydroxid dem Malonsäurediethylester zufügt, Malonsäurediethylester und Kaliumhydroxid in einem molaren Verhältnis von mindestens 1,5 anwendet und das Kaliumhydroxid in dem Malonsäurediethylester wirkungsvoll verteilt.

2. Verfahren nach Anspruch 1, **dadurch gekennzeichnet, daß** das Kaliumhydroxid in Form einer ethanolischen Lösung eingesetzt wird die gegebenenfalls noch andere, inerte niedrigsiedende Lösemittel enthält.

3. Verfahren nach einem der Ansprüche 1 oder 2, **dadurch gekennzeichnet, daß** die selektive Verseilung bei einer Temperatur von <80°C durchgeführt wird.

4. Verfahren nach einem der Ansprüche 1 oder 2, **dadurch gekennzeichnet, daß** die selektive Verseifung bei einer Temperatur von <60°C durchgeführt wird.

5. Verfahren nach einem der Ansprüche 1 oder 2, **dadurch gekennzeichnet, daß** die selektive Verseifung bei einer Temperatur von 0 bis 30°C durchgeführt wird.

6. Verfahren nach einem der Ansprüche 1 bis 5, **dadurch gekennzeichnet, daß** Malonsäurediethylester und Kaliumhydroxid im Molverhältnis von 1.5 bis 12 verwendet werden.

7. Verfahren nach einem der Ansprüche 1 bis 5, **dadurch gekennzeichnet, daß** Malonsäurediethylester und Kaliumhydroxid im Molverhältnis von 2 bis 8 verwendet werden.

8. Verfahren nach einem der Ansprüche 1 bis 5, **dadurch gekennzeichnet, daß** Malonsäurediethylester und Kaliumhydroxid im Molverhältnis von 3 bis 5 verwendet werden.

9. Verfahren nach einem der Ansprüche 1 bis 5, **dadurch gekennzeichnet, daß** die nach Abtrennung des festen Kaliummonoethylmalonats erhaltene Mutterlauge sowie die Waschflüssigkeit nach Abtrennung des gebildeten Alkohols und zusätzlich in die Reaktion eingebrachter inerter, niedrigsiedender Lösemittel und nach Ergänzung des verbrauchten Malonsäurediethylesters als Edukt und Reaktionsmedium in das Verfahren zurückgeführt werden.

10. Verfahren nach einem der Ansprüche 1 bis 9, **dadurch gekennzeichnet, daß** das Kaliumhydroxid in einer alkoholischen Lösung und der Malonsäurediethylester in Substanz verwendet werden, wobei der Alkohol in der 3- bis 10-fachen Gewichtsmenge, bezogen auf Kaliumhydroxid, eingesetzt wird.

## Claims

1. A process for preparing potassium monoethyl malonate by selective saponification of malonic acid diethyl ester with potassium hydroxide, **characterized in that** the potassium hydroxide is added to the malonic acid diethyl ester, using malonic acid diethyl ester and potassium hydroxide in a molar ratio of at least 1.5 and the potassium hydroxide is distributed effectively in the malonic acid diethyl ester.

2. A process according to claim 1, **characterized in that** the potassium hydroxide is used in the form of an ethanolic solution which, if desired, further contains other inert low-boiling solvents.

3. A process according to either of claims 1 or 2, **characterized in that** the selective saponification is carried out at a temperature of <80°C.

4. A process according to either of claims 1 or 2, **characterized in that** the selective saponification is carried out at a temperature of <60°C.

5. A process according to either of claims 1 or 2, **characterized in that** the selective saponification is carried out at a temperature of 0 to 30°C.

6. A process according to any one of claims 1 to 5, **characterized in that** malonic acid diethyl ester and potassium hydroxide are used in a molar ratio of 1.5 to 12.

7. A process according to any one of claims 1 to 5, **characterized in that** malonic acid diechyl ester and potassium hydroxide are used in a molar ratio of 2 to 8.

8. A process according to any one of claims 1 to 5, **characterized in that** malonic acid diethyl ester and potassium hydroxide are used in a molar ratio of 3 to 5.

9. A process according to any one of claims 1 to 5, **characterized in that** the mother liquor obtained after removal of the solid potassium monoethyl malonate and the washing liquid, after separating off the alcohol formed and inert low-boiling solvents additionally introduced into the reaction, and after supplementation of the malonic acid diethyl ester consumed, are recycled to the process as starting material and reaction medium.

10. A process according to any one of claims 1 to 9, **characterized in that** the potassium hydroxide is used in an alcoholic solution and the malonic acid diethyl ester is used without added solvent or diluent, the alcohol being used in 3 to 10 times the amount by weight, based on potassium hydroxide.

## Revendications

1. Procédé de préparation de malonate monoéthylique potassique par saponification sélective de l'ester diéthylique d'acide malonique avec de l'hydroxyde de potassium,
**caractérisé en ce qu'**
on ajoute l'hydroxyde de potassium au diester éthylique d'acide maionique, on utilise l'ester diéthylique d'acide malonique et l'hydroxyde de potassium dans un rapport molaire d'au moins 1,5 et qu'on répartit efficacement l'hydroxyde de potassium dans l'ester diéthylique d'acide malonique,

2. Procédé selon la revendication 1,
**caractérisé en ce qu'**
on met en oeuvre l'hydroxyde de potassium sous la forme d'une solution éthanolique qui contient éventuellement encore d'autres solvants inertes, à bas point d'ébullition.

3. Procédé selon l'une quelconque des revendications 1 ou 2,
**caractérisé en ce qu'**
on effectue la saponification sélective à une température de < 80°C.

4. Procédé selon l'une quelconque des revendications 1 ou 2,
**caractérisé en ce qu'**
on effectue la saponification sélective à une température de < 60°C.

5. Procédé selon l'une quelconque des revendications 1 ou 2,
**caractérisé en ce qu'**
on effectue la saponification sélective à une température allant de 0 à 30°C.

6. Procédé selon l'une quelconque des revendications 1 à 5,
**caractérisé en ce qu'**
on utilise l'ester diéthylique d'acide malonique et l'hydroxyde de potassium dans un rapport molaire allant de 1,5 à 12.

7. Procédé selon l'une quelconque des revendications 1 à 5,
**caractérisé en ce qu'**
on utilise l'ester diéthylique d'acide malonique et l'hydroxyde de potassium dans un rapport molaire allant de 2 à 8.

8. Procédé selon l'une quelconque des revendications 1 à 5,
**caractérisé en ce qu'**
on utilise l'ester diéthylique d'acide malonique et l'hydroxyde de potassium dans un rapport molaire allant de 3 à 5,

9. Procédé selon l'une quelconque des revendications 1 à 5,
**caractérisé en ce qu'**
en tant qu'éduit et milieu de réaction, on ramène dans le procédé les eaux mères obtenues après séparation du malonate monoéthylique potassique solide ainsi que les liquides de lavage après séparation de l'alcool formé, et en supplément des solvants à bas point d'ébullition, inertes, introduits dans la réaction, et après complément de l'ester diéthylique de l'acide malonique consommé.

10. Procédé selon l'une quelconque des revendications 1 à 9,
**caractérisé en ce qu'**
on utilise l'hydroxyde de potassium dans une solution alcoolique et l'ester diéthylique d'acide malonique en substance, l'alcool représentant 3 à 10 foil la quantité pondérale de l'hydroxyde de potassium.
